# NEUE EUROPÄISCHE PATENTSCHRIFT

(11) **EP 3 325 013 B2**
(45) Veröffentlichungstag und Bekanntmachung des Hinweises auf die Entscheidung über den Einspruch: **19.07.2023**
(45) Hinweis auf die Patenterteilung: 23.09.2020
(21) Anmeldenummer: 16739146.5
(22) Anmeldetag: 15.07.2016
(51) Int. Cl.: A61K 45/06, A61K 31/216, A61K 31/41, A61K 31/506, A61K 31/519, A61P 9/00, A61P 3/14, A61P 7/02, A61P 7/10, A61P 9/04, A61P 11/00, A61P 13/12, A61P 43/00, A61P 3/06

(54) **STIMULATOREN UND/ODER AKTIVATOREN DER LÖSLICHEN GUANYLATZYKLASE (SGC) IN KOMBINATION MIT EINEM INHIBITOR DER NEUTRALEN ENDOPEPTIDASE (NEP INHIBITOR) UND EINEM ANGIOTENSIN AII-ANTAGONISTEN UND IHRE VERWENDUNG**
STIMULATORS / ACTIVATORS OF SOLUBLE GUANYLAT CYCLASE IN COMBINATION WITH A NEP-INHIBITOR AND AN ANGIOTENSIN AII-ANTAGONIST AND THE USE THEREOF
STIMULATEURS / ACTIVATEURS DE GUANYLATE CYCLASE SOLUBLE EN COMBINAISON AVEC UN INHIBITEUR DE L'ENDOPEPTIDASE NEUTRALE (NEP) ET UN ANTAGONIST D'ANGIOTENSIN II ET SON UTILISATION

(30) Priorität: 23.07.2015 EP 15178141
(43) Veröffentlichungstag der Anmeldung: 30.05.2018
(73) Patentinhaber: Bayer Pharma Aktiengesellschaft, 13353 Berlin (DE)
(72) Erfinder: MARQUARDT, Tobias, 42115 Wuppertal (DE); FOLLMANN, Markus, 50859 Köln (DE); STASCH, Johannes-Peter, 00046 Grottaferrata (RM) (IT)
(74) Vertreter: König Szynka Tilmann von Renesse Patentanwälte Partnerschaft mbB Düsseldorf
(86) Internationale Anmeldenummer: PCT/EP2016/066891
(87) Internationale Veröffentlichungsnummer: WO 2017/013010

(56) Entgegenhaltungen:
- WO-A1-2011/147809
- WO-A1-2012/139888
- WO-A1-2016/087342
- WO-A2-2011/056511
- JOHN J.V. MCMURRAY ET AL: "Angiotensin-Neprilysin Inhibition versus Enalapril in Heart Failure", NEW ENGLAND JOURNAL OF MEDICINE, Bd. 371, Nr. 11, 11. September 2014 (2014-09-11), Seiten 993-1004, XP055175908, ISSN: 0028-4793, DOI: 10.1056/NEJMoa1409077
- M. KOMAJDA ET AL: "Heart failure with preserved ejection fraction: a clinical dilemma", EUROPEAN HEART JOURNAL, Bd. 35, Nr. 16, 11. März 2014 (2014-03-11) , Seiten 1022-1032, XP055305898, GB ISSN: 0195-668X, DOI: 10.1093/eurheartj/ehu067
- BEYER C ET AL: "Stimulation of the soluble guanylate cyclase (sGC) inhibits fibrosis by blocking non-canonical TGF[beta] signalling", ANNALS OF THE RHEUMATIC DISEASES, BRITISH MEDICAL ASSOCIATION, GB, vol. 74, no. 7, 1 July 2015 (2015-07-01), pages 1408-1416, XP009509498, ISSN: 0003-4967, DOI: 10.1136/ANNRHEUMDIS-2013-204508 [retrieved on 2014-02-23]
- J.-P. Stasch ET AL: "Soluble Guanylate Cyclase as an Emerging Therapeutic Target in Cardiopulmonary Disease", Circulation, vol. 123, no. 20, 23 May 2011 (2011-05-23) , pages 2263-2273, XP055440845, US ISSN: 0009-7322, DOI: 10.1161/CIRCULATIONAHA.110.981738
- Johannes-Peter Stasch ET AL: "Renal effects of soluble guanylate cyclase stimulators and activators: A review of the preclinical evidence", CURRENT OPINION IN PHARMACOLOGY, vol. 21, 1 April 2015 (2015-04-01), pages 95-104, XP055545669, NL ISSN: 1471-4892, DOI: 10.1016/j.coph.2014.12.014

## Beschreibung

Die vorliegende Erfindung betrifft die Kombinationen gemäß Anspruch 1 und deren Anwendung zur Behandlung und/oder Prophylaxe von Herz-Kreislauf-Erkrankungen z.B. Herzinsuffizienz mit erhaltener Ejektionsfraktion oder Herzinsuffizienz mit reduzierter Ejektionsfraktion, renalen Erkrankungen z.B. chronisches Nierenversagen, urologischen Erkrankungen, Lungenerkrankungen, Erkrankungen des Zentralnervensystems, zur Regulation der cerebralen Durchblutung z.B. bei vaskulären cerebralen Demenzzuständen, zur Behandlung und/oder Prophylaxe von fibrotischen Erkrankungen sowie anderen Krankheitserscheinungen (z.B. Endorganschäden, die Hirn, Niere oder Herz betreffen).

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatzyklasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatzyklasen und die löslichen, durch NO stimulierbaren Guanylatzyklasen. Die löslichen Guanylatzyklasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO. Die partikulären, membranständigen Guanylatzyklasen bestehen aus der zytosolischen katalytischen Domäne, einer Transmembranregion und der extrazellulären Liganden-bindenden Domäne. Die Bindung natriuretischer Peptide an die extrazelluläre Liganden-bindenden Domäne führt zur Aktivierung der katalytischen Domäne und die Biosynthese von cGMP aus GTP. Neutrale Endopeptidase (Neprilysin) inaktiviert natriuretische Peptide durch proteolytische Spaltung und wirkt folglich inhibierend auf die partikuläre Guanylatzyklase.

Durch die Bildung von cGMP und der daraus resultierenden Regulation von Phosphodiesterasen, Ionenkanälen und Proteinkinasen spielt die Guanylatzyklase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Arteriosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatzyklase wurden bisher überwiegend Verbindungen wie organische Nitrate verwendet, deren Wirkung auf direkte Freisetzung von NO beruht. Dieses wird durch Biokonversion gebildet und aktiviert die lösliche Guanylatzyklase durch Angriff am Eisen-Zentralatom des Häms. Neben den Nebenwirkungen gehört die Toleranzentwicklung zu den entscheidenden Nachteilen dieser Behandlungsweise.

Durch sGC-Aktivatoren und Stimulatoren werden die native wie auch die Häm-freie Formen der löslichen Guanylatzyklase direkt aktiviert bzw. stimuliert.

Durch sGC Aktivatoren ist es möglich, auch oxidierte Formen der löslichen Guanylatzyklase und letztendlich auch die Häm-freie Form der sGC unabhängig von NO direkt zu stimulieren. Diese oxidierte/Häm-freie Form könnte sich in Geweben, die oxidativem Stress ausgesetzt sind, in höheren Konzentrationen anreichern, so dass es durch den Einsatz von sGC Aktivatoren auch zu einer gerichteten Behandlung von Geweben, die unter oxidativem Stress stehen, kommen sollte.

LCZ696 ist ein ARNI (Angiotensin-Rezeptor-Neprilysin-Inhibitor) und somit ein dualer Wirkstoff, der aus dem Angiotensin All-Antagonisten Valsartan und dem Neprilysin-Inhibitor Sacubitril besteht. Durch die Neprilysin Inhibition erreicht man eine verminderte Degradation von natriuretischen Peptiden. Diese wirken v.a. diuretisch und natriuretisch durch ihre gefäßdilatierende Wirkung auf präglomeruläre Gefäße. Darüber hinaus können sie auch die Natriumrückresorption in proximalen Tubulusabschnitten inhibieren.

Die Kombination von Angiotensinrezeptor-Blockade und Neprilysin-Inhibition durch LCZ696 (einer Kombination des Angiotensinrezeptor Antagonisten Valsartan und des NEP-Inhibitors Sacubitril) wurde kürzlich in der klinischen Erprobung (Phase III) bei Patienten mit Herzinsuffizienz untersucht und führte zu einer Reduktion des Todes- und Hospitalisierungsrisikos (McMurray et al 2014 NEJM). Außer der gewünschten ANP- und cGMP-Erhöhung wurde unter LCZ696 Gabe sowohl in gesunden Probanden als auch in hypertensiven Patienten ein kompensatorischer Anstieg von Renin und Angiotensin gemessen (Gu J. et al J Clin Pharmacol. 2010 Apr;50(4):401-14).

Ein Nachteil bei der Verabreichung von LCZ696 zur Blutdurcksenkung ist, dass kompensatorische Effekte der Herzfrequenz wie bspw. eine Reflextachykardie mit einhergehender Blutdrucksenkung zu beobachten sind.

Die Aufgabe der vorliegenden Erfindung besteht demnach in der Bereitstellung von Kombinationen pharmazeutischer Wirkstoffe zur Behandlung von Herz-Kreislauf-Krankheiten, welche den mittleren arteriellen Blutdruck senken und möglichst keinen oder wenig Effekte auf die hämodynamischen Parameter, wie die Herzrate haben. Damit sollen die oben beschriebenen Nachteile, der kompensatorischen Effekte der Herzfrequenz, die mit einer Blutdrucksenkung einhergehen, überwunden werden.

Zur Lösung dieser Aufgabe wurden sGC Stimulatoren und/oder sGC Aktivatoren in Kombination mit Neprilysin Inhibitoren und/oder Angiotensin All-Antagonisten unter akuten und insbesondere unter chronischem Einsatz in der Annahme untersucht, dass positive Effekte auf Blutdruck und Herzfrequenz unter experimentellen Bedingungen gezeigt werden können, die durch die resultierenden Plasma und Gewebe cGMP Spiegel hervorgerufen werden. Diese experimentellen Bedingungen bestehen aus gesunden Tieren oder auch Tieren mit Bluthochdruck (z.B. spontan hypertensive Ratten). Hierbei werden die Experimente mit sGC Stimulatoren und/oder Aktivatoren "head to head" gegen die alleinige Kombination aus Neprilysin Inhibitoren und Angiotensin All-Antagonisten, wie bspw. LCZ696 durchgeführt.

Diese Experimente sollen Aufschluss darüber geben, ob die Erhöhung von cGMP durch Stimulation der löslichen Guanylatzyklase mit sGC Stimulatoren und/oder Aktivatoren in Kombination mit Sacubitril (Aktivierung der partikulären, membranständigen Guanylatzyklase durch Inhibition von Neprilysin) und/oder einem Angiotensin All-Antagonisten eine vorteilhafte Wirkung auf z.B. hämodynamische Parameter wie die Herzrate und den mittleren arteriellen Blutdruck bewirkt.

Die Lösung der oben gestellten Aufgabe und Gegenstand der vorliegenden Erfindung sind die im Folgenden genannten Kombinationen aus dem sGC Stimulator der Formel (6) mit Sacubitril und Valsartan.

Die erfindungsgemäße Kombination führt zu einer Gefäßrelaxation und/oder kontrollierbaren Blutdrucksenkung. Die Kombination eignet sich daher zur Behandlung und/oder Prophylaxe von Krankheiten, vorzugsweise von kardiovaskulären Erkrankungen, insbesondere zur Behandlung und/oder Prophylaxe von Herzinsuffizienz mit erhaltener Ejektionsfraktion oder Herzinsuffizienz mit reduzierter Ejektionsfraktion, renalen Erkrankungen, Lungenerkrankungen, sowie zur Behandlung und/oder fibrotischen Erkrankungen bei Menschen und Tieren. Der Schutzumfang ist durch die Ansprüche definiert.

Angiotensin All-Antagonisten der vorliegenden offenbarten Kombinationen sind beispielhaft und vorzugsweise Valsartan, Losartan, Candesartan, Telmisartan, Irbesartan, Olmesartan, Eprosartan oder Azilsartan und vorzugsweise Valsartan.

Valsartan ist der Angiotensin All-Antagonist (S)-N-(1-Carboxy-2-methylprop-1-yl)-N-pentanoyl-N-[2'-(1H-tetrazol-5-yl)biphenyl-4-yl-methyl]amin der Formel (30) 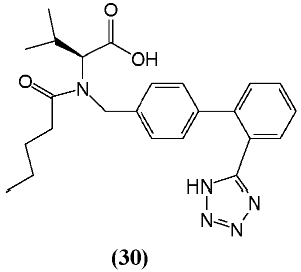 oder ein Salz, Solvat oder Solvat der Salze davon und ist in EP 0 443 983 A und US 5 399 578 A beschrieben.

Der NEP-Inhibitor der vorliegenden erfindungsgemäßen Kombinationen ist Sacubitril der Formel (31) 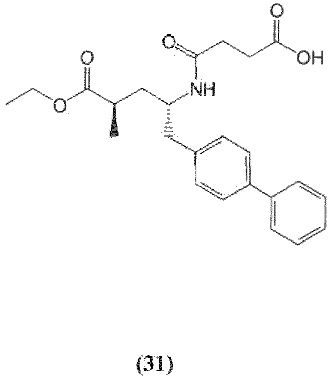 und ist in EP005551751 beschrieben.

In Bezug auf Sacubitril umfassen bevorzugte Salze das Natriumsalz, das Triethanolaminsalz und das Tris(hydroxymethyl)aminomethansalz.

In den erfindungsgemäßen Kombinationen können Valsartan und der NEP-Inhibitor jeweils einzeln oder als Trinatrium[3-((1*S*,3*R*)-1-biphenyl-4-ylmethyl-3-ethoxycarbonyl-1-butylcarbamoyl)propionat-(*S*)-3'-methyl-2'-(pentanoyl{2"-(tetrazol-5-ylat)biphenyl-4'-ylmethyl}amino)butyrat]hemipentahydrat vorliegen.

Der Trinatrium[3-((1*S*,3*R*)-1-biphenyl-4-ylmethyl-3-ethoxycarbonyl-1-butylcarbamoyl)propionat-(*S*)-3'-methyl-2'-(pentanoyl{2"-(tetrazol-5-ylat)biphenyl-4'-ylmethyl}amino)butyrat]hemipentahydrat Komplex, auch bekannt als LCZ696, ist näher in EP 1 948 158 A1 beschrieben.

In den erfindungsgemäßen Kombinationen ist der sGC Stimulator die Verbindung der Formel (6).

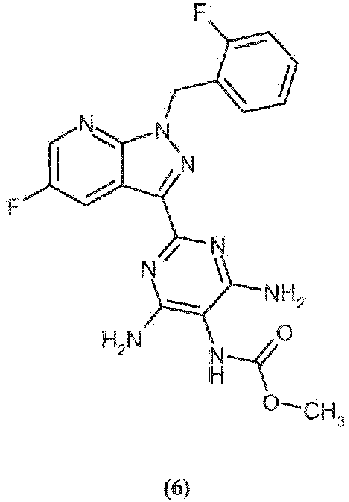

Die erfindungsgemäßen Kombinationen erlauben eine effektive Behandlung von Herz-Kreislauf-Krankheiten, indem der mittlere arterielle Blutdruck gesenkt wird und möglichst keine oder wenig Effekte auf die hämodynamischen Parameter, wie die Herzrate auftreten. Damit konnten die oben beschriebenen Nachteile der im Stand der Technik bekannten Therapieformen, wie bspw. kompensatorische Effekte der Herzfrequenz mit einhergehender Blutdrucksenkung, überwunden werden.

Darüber hinaus zeigen die erfindungsgemäßen Kombinationen ein nicht vorhersehbares, wertvolles pharmakologisches und pharmakokinetisches Wirkspektrum.

Die erfindungsgemäßen Kombinationen sind aufgrund ihrer gefäßrelaxierender Wirkung (Gefäßrelaxation) und Hemmung der Thrombozytenaggregation geeignet für die Prophylaxe und/oder Behandlung von Krankheiten und führen zu einer Blutdrucksenkung sowie zu einer Steigerung des koronaren Blutflusses. Diese Wirkungen sind über eine Stimulation der löslichen und/oder partikulären Guanylatzyklase und/oder einer Blockade des Angiotensinrezeptors vermittelt. Außerdem verstärken die erfindungsgemäßen Kombinationen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Ein weiterer Gegenstand der vorliegenden Erfindung ist die Anwendung von der erfindungsgemäßen Kombination zur Behandlung von Herz-Kreislauf-Erkrankungen z.B. Herzinsuffizienz mit erhaltener Ejektionsfraktion oder Herzinsuffizienz mit reduzierter Ejektionsfraktion, renalen Erkrankungen z.B. chronisches Nierenversagen, urologischen Erkrankungen, Lungenerkrankungen, Erkrankungen des Zentralnervensystems, zur Regulation der cerebralen Durchblutung z.B. bei vaskulären cerebralen Demenzzuständen, zur Behandlung und/oder Prophylaxe von fibrotischen Erkrankungen sowie anderen Krankheitserscheinungen (z.B. Endorganschäden, die Hirn, Niere oder Herz betreffen).

Ein weiterer Gegenstand der vorliegenden Erfindung ist die erfindugsgemäße Kombination sowie deren Anwendung zur Behandlung von Herz-Kreislauf-Erkrankungen z.B. Herzinsuffizienz mit erhaltener Ejektionsfraktion oder Herzinsuffizienz mit reduzierter Ejektionsfraktion, renalen Erkrankungen z.B. chronisches Nierenversagen, urologischen Erkrankungen, Lungenerkrankungen, Erkrankungen des Zentralnervensystems, zur Regulation der cerebralen Durchblutung z.B. bei vaskulären cerebralen Demenzzuständen, zur Behandlung und/oder Prophylaxe von fibrotischen Erkrankungen sowie anderen Krankheitserscheinungen (z.B. Endorganschäden, die Hirn, Niere oder Herz betreffen).

Gegenstand der Erfindung ist der sGC Stimulator der Formel (6) in Kombination mit Sacubitril Valsartan verabreicht.

Gegenstand der vorliegenden Erfindung sind Kombinationen enthaltend den sGC Stimulator, Sacubitril und Valsartan sowie jeweils die Salze, Solvate und Solvate der Salze davon.

Ein weiterer bevorzugter Gegenstand der vorliegenden Erfindung sind Kombinationen enthaltend den sGC Stimulator und LCZ696 sowie jeweils die Salze, Solvate und Solvate der Salze davon.

Ein besonders bevorzugter Gegenstand der vorliegenden Erfindung sind Kombinationen enthaltend den sGC Stimulator, Sacubitril und Valsartan sowie jeweils die Salze, Solvate und Solvate der Salze davon.

Ein weiterer besonders bevorzugter Gegenstand der vorliegenden Erfindung sind Kombinationen enthaltend den sGC Stimulator und LCZ696 sowie jeweils die Salze, Solvate und Solvate der Salze davon.

Gegenstand der vorliegenden Erfindung sind Kombinationen enthaltend die Verbindung der Formel (6), Sacubitril und Valsartan, sowie jeweils die Salze, Solvate und Solvate der Salze von der Verbindung der Formel (6) und Sacubitril und Valsartan.

Ein weiterer besonders bevorzugter Gegenstand der vorliegenden Erfindung sind Kombinationen enthaltend die Verbindung der Formel (6) und LCZ696 sowie jeweils die Salze, Solvate und Solvate der Salze davon.

Die zu kombinierenden Komponenten können als Salze vorliegen. Als Salze sind im Rahmen der vorliegenden Erfindung physiologisch unbedenkliche Salze derzu kombinierenden Verbindungen bevorzugt. Umfasst sind auch Salze, die für pharmazeutische Anwendungen selbst nicht geeignet sind, jedoch beispielsweise für die Isolierung oder Reinigung der zu kombinierenden Verbindungen verwendet werden können.

Ein weiterer Gegenstand der vorliegenden Erfindung sind Kombinationen, in denen das molare Verhältnis der Verbindung der Formel (6) zu Valsartan zu Sacubitril 0.01-1:1:1 ist.

Ein weiterer Gegenstand der vorliegenden Erfindung sind Kombinationen, in denen das molare Verhältnis der Verbindung der Formel (6) zu Trinatrium-[3-((1*S*,3*R*)-1-biphenyl-4-ylmethyl-3-ethoxycar-bonyl-1-butylcarbamoyl)propi-onat-(*S*)-3'-methyl-2'-(pentanoyl{2"-(tetrazol-5-ylat)biphenyl-4'-ylmethyl}amino)butyrat]-hemipentahydrat 0.01-1:1 ist.

Ein weiterer Gegenstand der vorliegenden Erfindung sind Kombinationen, in denen das molare Verhältnis der Verbindung der Formel (6) zu Sacubitril 0.01-1:1 ist.

Ein weiterer Gegenstand der vorliegenden Erfindung sind Kombinationen, in denen das molare Verhältnis der Verbindung der Formel (6) zu Valsartan 0.01-1:1 ist.

Ein weiterer Gegenstand der vorliegenden Erfindung sind erfindungsgemäße Kombinationen zur Behandlung und/oder Prophylaxe von Krankheiten, wobei die Verbindung der Formel (6) einmal täglich und Valsartan und Sacubitril zweimal täglich verabreicht werden.

Ein weiterer Gegenstand der vorliegenden Erfindung sind erfindungsgemäße Kombinationen zur Behandlung und/oder Prophylaxe von Krankheiten, wobei 1.25-20 mg der Verbindung der Formel (6), 20-200 mg Valsartan und 20-200 mg Sacubitril verabreicht werden.

Ein weiterer Gegenstand der vorliegenden Erfindung sind erfindungsgemäße Kombinationen zur Behandlung und/oder Prophylaxe von Krankheiten, wobei 1.25-20 mg der Verbindung der Formel (6) und 20-200 mg Sacubitril verabreicht werden.

Ein weiterer Gegenstand der vorliegenden Erfindung sind erfindungsgemäße Kombinationen zur Behandlung und/oder Prophylaxe von Krankheiten, wobei 1.25 - 20 mg der Verbindung der Formel (6) und 20-200 mg Valsartan verabreicht werden

Ein weiterer Gegenstand der vorliegenden Erfindung sind erfindungsgemäße Kombinationen zur Behandlung und/oder Prophylaxe von Krankheiten, wobei die Verbindung der Formel (6) einmal täglich verabreicht wird und Trinatrium-[3-((1*S*,3*R*)-1-biphenyl-4-ylmethyl-3-ethoxycar-bonyl-1-butylcarbamoyl)propionat-(*S*)-3'-methyl-2'-(pentano-yl{2"-(tetrazol-5-ylat)biphenyl-4'-ylmethyl}amino)butyrat]-hemipentahydrat zweimal täglich verabreicht wird.

Ein weiterer Gegenstand der vorliegenden Erfindung sind erfindungsgemäße Kombinationen zur Behandlung und/oder Prophylaxe von Krankheiten, wobei 1.25-20 mg der Verbindung der Formel (6) und 40-400 mg Trinatrium-[3-((1*S*,3*R*)-1-biphenyl-4-ylmethyl-3-ethoxycar-bonyl-1-butylcarbamoyl)propionat-(*S*)-3'-methyl-2'-(pentano-yl{2"-(tetrazol-5-ylat)biphenyl-4'-ylmethyl}amino)butyrat]-hemipentahydrat verabreicht werden.

Ein weiterer Gegenstand der vorliegenden Erfindung sind erfindungsgemäße Kombinationen, worin das molare Verhältnis der Verbindung der Formel (6) zu Valsartan zu Sacubitril 0.001-1:1:3, 0.001-1:3:1, 0.001-1:1:2, 0.001-1:2:1 oder 0.001-1:1:1, vorzugsweise 0.005-0.75:1.3, 0.005-0.75:3:1,0.005-0.75:1:2, 0.005-0.75:2:1 oder 0.005-0.75:1:1 und am meisten bevorzugt 0.01-0.5:1:1 ist.

Ein weiterer Gegenstand der vorliegenden Erfindung sind erfindungsgemäßen Kombinationen, worin das molare Verhältnis von der Verbindung der Formel (6) zu Trinatrium-[3-((1*S*,3*R*)-1-biphenyl-4-ylmethyl-3-ethoxycar-bonyl-1-butylcarbamoyl)propionat-(*S*)-3'-methyl-2'-(pentanoyl{2"-(tetrazol-5-ylat)biphenyl-4'-ylmethyl}amino)butyrat]-hemi-pentahydrat 0.001-1:1, vorzugsweise 0.005-0.75:1 und am meisten bevorzugt 0.01-0.5:1 ist.

Ein weiterer Gegenstand der vorliegenden Erfindung sind die erfindungsgemäßen Kombinationen zur Behandlung und/oder Prophylaxe von Krankheiten.

Ein weiterer Gegenstand der vorliegenden Erfindung sind die erfindungsgemäßen Kombinationen zur Verwendung in einem Verfahren zur Behandlung und/oder Prophylaxe von Herz-Kreislauf-Erkrankungen, renalen Erkrankungen, Lungenerkrankungen, sowie zur Behandlung und/oder Prophylaxe von fibrotischen Erkrankungen.

Ein weiterer Gegenstand der vorliegenden Erfindung ist ein Arzneimittel enthaltend mindestens eine erfindungsgemäße Kombination in Kombination mit einem inerten, nicht-toxischen, pharmazeutisch geeigneten Hilfsstoff.

Ein weiterer Gegenstand der vorliegenden Erfindung ist ein Arzneimittel enthaltend mindestens eine erfindungsgemäße Kombination in Kombination mit einem oder mehreren weiteren Wirkstoffen ausgewählt aus der Gruppe bestehend aus ACE-Inhibitoren, Renin-Inhibitoren, Beta-Blocker, Acetylsalicylsäure, Diuretika, Calcium-Antagonisten, Statine, Digitalis (Digoxin)-Derivate, Calcium-Sensitizer, Nitrate sowie Antithrombotika.

Ein weiterer Gegenstand der vorliegenden Erfindung ist ein Arzneimittel enthaltend mindestens eine erfindungsgemäße Kombination zur Behandlung und/oder Prophylaxe von Herz-Kreislauf-Erkrankungen, renalen Erkrankungen, Lungenerkrankungen, sowie zur Behandlung und/oder Prophylaxe von fibrotischen Erkrankungen.

Ein weiterer Gegenstand der vorliegenden Erfindung ist Verfahren zur Behandlung und/oder Prophylaxe von Herz-Kreislauf-Erkrankungen, renalen Erkrankungen, Lungenerkrankungen, sowie zur Behandlung und/oder Prophylaxe von fibrotischen Erkrankungen in Menschen und Tieren unter Verwendung mindestens einer erfindungsgemäßen Kombination.

Die Erfindung betrifft auch die Kombination von getrennten pharmazeutischen Zusammensetzungen in Kitform. Dies ist ein Kit, das zwei oder drei getrennte Einheiten umfasst: Eine pharmazeutische Zusammensetzung des sGC Stimulators, einer pharmazeutischen Sacubitril zussamensetzung und einer pharmazeutischen Valsartanzusammensetzung.

Die Erfindung betrifft außerdem eine bevorzugte Kitform, die zwei Einheiten umfasst: Eine pharmazeutische Zusammensetzung enthaltend den sGC Stimulator und eine pharmazeutische Zusammensetzung enthaltend Sacubitril und Valsartan.

Die Erfindung betrifft außerdem eine bevorzugte Kitform, die zwei Einheiten umfasst: Eine pharmazeutische Zusammensetzung enthaltend den sGC Stimulator und eine pharmazeutische Zusammensetzung enthaltend LCZ696.

Die Erfindung betrifft außerdem eine bevorzugte Kitform, die zwei Einheiten umfasst: Eine pharmazeutische Zusammensetzung umfassend die Verbindung der Formel (6) und eine pharmazeutische Zusammensetzung umfassend LCZ696.

Das Kit ist insbesondere vorteilhaft, wenn die getrennten Komponenten in unterschiedlichen Dosisformen verabreicht werden müssen oder in unterschiedlichen Dosisintervallen verabreicht werden.

Ein weiterer Gegenstand der vorliegenden Erfindung ist ein Kit umfassend eine pharmazeutische Zusammensetzung enthaltend einen sGC Stimulator und eine pharmazeutische Zusammensetzung enthaltend den Angiotensin AII Antagonisten und Sacubitril.

Ein weiterer Gegenstand der vorliegenden Erfindung ist ein Kit umfassend eine pharmazeutische Zusammensetzung enthaltend einen sGC Stimulator und eine pharmazeutische Zusammensetzung enthaltend Valsartan und Sacubitril.

Ein weiterer Gegenstand der vorliegenden Erfindung ist ein Kit umfassend eine pharmazeutische Zusammensetzung enthaltend den sGC Stimulator und eine pharmazeutische Zusammensetzung enthaltendTrinatrium-[3-((1S,3R)-1-biphenyl-4-ylmethyl-3-ethoxycar-bonyl-1-butylcarbamoyl)propionat-(S)-3'-methyl-2'-(pentanoyl{2"-(tetrazol-5-ylat)biphenyl-4'-ylmethyl}amino)butyrat]-hemipentahydrat.

Ein weiterer Gegenstand der vorliegenden Erfindung ist ein Kit umfassend eine pharmazeutische Zusammensetzung enthaltend die Verbindung der Formel (6) und eine pharmazeutische Zusammensetzung enthaltend Trinatrium-[3-((1*S*,3*R*)-1-biphenyl-4-ylmethyl-3-ethoxycar-bonyl-1-butylcarbamoyl)propionat-(*S*)-3'-methyl-2'-(pentano-yl{2"-(tetrazol-5-ylat)biphenyl-4'-ylmethyl}amino)butyrat]-hemipentahydrat.

Die erfindungsgemäßen Kombinationen können daher in Arzneimitteln zur Behandlung und/oder Prophylaxe von kardiovaskulären Erkrankungen wie beispielsweise Bluthochdruck (Hypertonie), resistente Hypertonie, akute und chronische Herzinsuffizienz, Herzinsuffizienz mit erhaltener Ejektionsfraktion (HFpEF) oder Herzinsuffizienz mit reduzierter Ejektionsfraktion (HFrEF) koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AVjunktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von männlicher erektiler Dysfunktion und weiblicher sexueller Dysfunktion eingesetzt werden.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz und akute Phasen der Verschlechterung einer bestehenden chronischen Herzinsuffizienz (worsening heart failure).

Darüber hinaus können die erfindungsgemäßen Kombinationen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Kombinationen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden. Die erfindungsgemäßen Kombinationen eignen sich auch zur Behandlung der Muskeldystrophie, wie der Muskeldystrophie Becker-Kiener (BMD) und Muskeldystrophie Duchenne (DMD).

Weiterhin eignen sich die erfindungsgemäßen Kombinationen zur Behandlung und/oder Prophylaxe urologischer Erkrankungen wie beispielsweise benignes Prostata-Syndrom (BPS), benigne Prostata-Hyperplasie (BPH), benigne Prostata Vergrößerung (BPE), Blasenentleerungsstörung (BOO), untere Harnwegssyndrome (LUTS, einschließlich Felines Urologisches Syndrom (FUS)), Erkrankungen des Urogenital-Systems einschließlich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Kombinationen zur Behandlung und/oder Prophylaxe von Nierenerkrankungen, insbesondere von akuter und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittel-induzierte Nephropathie, diabetische und nichtdiabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Kombinationen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignet sich die erfindungsgemäßen Kombinationen auch zur Behandlung und/oder Prophylaxe von asthmatischen Erkrankungen, Lungenerkrankungen wie z.B. pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und derzystischen Fibrose (CF). Außerdem können die genannten erfindungsgemäßen Kombinationen als Bronchodilatatoren eingesetzt werden.

Die in der vorliegenden Erfindung beschriebenen Kombinationen stellen auch Kombinationen zur Behandlung und/oder Prophylaxe von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentralnervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Kombinationen auch zur Regulation der cerebralen Durchblutung und stellen beispielsweise wirkungsvolle Mittel zur Bekämpfung vascular cerebraler Demenzzustände und von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Kombinationen zur Bekämpfung von Schmerzzuständen und Tinnitus eingesetzt werden.

Zudem besitzen die erfindungsgemäßen Kombinationen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Desweiteren können die erfindungsgemäßen Kombinationen ebenfalls zur Behandlung und/ oder Prophylaxe von Autoimmunerkrankungen eingesetzt werden.

Weiterhin können die erfindungsgemäßen Kombinationen zur Behandlung und/oder Prophylaxe fibrotischer Erkrankungen der inneren Organe, wie beispielsweise der Lunge, des Herzens, der Niere, der Haut, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, systemische Sklerose, Sklerodermie, digitale Ulzerationen, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Kombinationen zur Bekämpfung postoperativer Narbenbildung, z.B. in Folge von Glaukom-Operationen.

Die erfindungsgemäßen Kombinationen können ebenfalls kosmetisch bei alternder und verhornender Haut eingesetzt werden.

Außerdem sind die erfindungsgemäßen Kombinationen zur Behandlung und/ oder Prophylaxe von Hepatitis, Neoplasma, Osteoporose, Knochenbildungsstörungen, Glaukom und Gastroparese geeignet.

Die erfindungsgemäßen Kombinationen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Kombinationen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- den Blutdrucksenkende Wirkstoffe, beispielhaft und vorzugsweise aus der Gruppe derAngiotensin All-Antagonisten, ACE-Hemmer, Calcium-Antagonisten, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika;
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2, 5 und/oder 9 insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Kombinationen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Kombinationen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der werden die erfindungsgemäßen Kombinationen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Kombinationen in Kombination mit einem Faktor Xa-Inhibitor, wie beispielhaft und vorzugsweise Rivaroxaban, DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Kombinationen in Kombination mit Heparin oder einem low molecular weight (LMW)-Heparin-Derivat verabreicht.

Bei einer bevorzugten Ausführungsform der Erfindung werden die erfindungsgemäßen Kombinationen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin All-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Kombinationen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Kombinationen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Kombinationen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Kombinationen in Kombination mit einem Angiotensin All-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Kombinationen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Kombinationen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Kombinationen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Kombinationen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Kombinationen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Kombinationen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Kombinationen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Kombinationen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Kombinationen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Kombinationen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Kombinationen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Kombinationen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Kombinationen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Kombinationen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Kombinationen in Kombination mit einem Lipase-Inhibitor, wie beispielhaft und vorzugsweise Orlistat, verabreicht.

Bei einer bevorzugten Ausführungsform der Erfindung werden die erfindungsgemäßen Kombinationen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Kombinationen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Kombinationen in Kombination mit einem Lipoprotein(a)-Antagonisten, wie beispielhaft und vorzugsweise Gemcabene calcium (CI-1027) oder Nicotinsäure, verabreicht.

In den erfindungsgemäßen Kombinationen können die Komponenten systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Kombinationen in geeigneten Applikationsformen verabreicht werden.

Für die orale Applikation eignen sich nach dem Stand der Technik funktionierende, die erfindungsgemäßen Kombinationen schnell und/oder modifiziert abgebende Applikationsformen, die die Verbindungen, welche Bestandteil der Kombination sind, in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der den erfindungsgemäßen Kombinationen zugrunde liegenden Verbindungen kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Als bevorzugte Applikationsformen sind zu nennen Tablettenform (nicht-überzogene oder überzogene Tabletten, beispielsweise mit magensaftresistenten oder sich verzögert auflösenden oder unlöslichen Überzügen, die die Freisetzung der den erfindungsgemäßen Kombinationen zugrunde liegenden Verbindungen kontrollieren), in der Mundhöhle schnell zerfallende Tabletten oder Filme/Oblaten und insbesondere bevorzugte Applikationsformen sind Tablettenform (nicht-überzogene oder überzogene Tabletten, beispielsweise mit magensaftresistenten oder sich verzögert auflösenden oder unlöslichen Überzügen, die die Freisetzung der den erfindungsgemäßen Kombinationen zugrunde liegenden Komponenten kontrollieren), in der Mundhöhle schnell zerfallende Tabletten oder Filme/Oblaten.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt ist die orale oder parenterale Applikation, wobei die orale Applikation bevorzugter ist. Insbesondere bevorzugt ist die orale Applikation mittels Tablettenform.

In den erfindungsgemäßen Kombinationen können die Komponenten in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

In den erfindungsgemäßen Kombinationen können die Komponenten zusammen oder nacheinander oder getrennt in einer kombinierten Einheitsdosierungsform, in zwei getrennten Einheitsdosierungsformen oder in drei getrennten Einheitsdosierungsformen verabreicht werden. Die Einheitsdosierungsform kann auch eine fixierte Kombination sein.

Eine therapeutisch wirksame Menge jeder Komponente der erfindungsgemäßen Kombination kann simultan oder sequenziell in jeder Reihenfolge verabreicht werden.

In einer Ausführungsform können die Komponenten in einer sogenannten Retard-Formulierung vorliegen, in der die Freisetzung der erfindungsgemäßen Komponenten zu unterschiedlichen Zeitpunkten stattfindet. Beispielsweise genannt sei eine Tablette mit sich verzögert auflösenden Überzügen, die jeweils eine oder mehrere Komponenten der erfindungsgemäßen Kombinationen enthält.

In einer Ausrührungsform der Erfindung beträgt bei oraler Applikation die Dosierung des sGC Stimulators etwa 1.25-20 mg, 1.25-5 mg od, etwa 5-10 mg od oder 10 bis 20 mg od.

In einer Ausführungsform der Erfindung beträgt die Dosierung von Valsartan etwa 20-110 mg bid, 20-50 mg bid, oder 50-110 mg bid.

In einer Ausführungsform der Erfindung beträgtdie Dosierung des NEP-Inhibitors etwa 20-100 mg bid, etwa 20-50 mg bid oder 50 bis 100 mg bid.

In einer Ausführungsform der Erfindung beträgt die Dosierung von LCZ696 etwa 40 - 400 mg, 50-200 mg bid, 50-100 mg bid oder 100-200 mg bid.

In einer Ausführungsform der Erfindung wird Valsartan in Form einer geeigneten Einheitsdosierungsform, beispielsweise einer Kapsel oder Tablette bereitgestellt und umfasst eine therapeutische wirksame Menge von beispielsweise 20 bis 320 mg an Valsartan, die Patienten verabreicht werden kann. Die Verabreichung des Wirkstoffs kann dreimal am Tag stattfinden, ausgehend beispielsweise von einer Tagesdosis von 20 mg oder 40 mg an Valsartan, was über 80 mg täglich und weiter auf 160 mg täglich bis 320 mg täglich ansteigt. Vorzugsweise wird Valsartan einmal am Tag oder zweimal am Tag bei Patienten mit Herzschwäche mit einer Dosis von jeweils 80 mg oder 160 mg verabreicht. Entsprechende Dosen können beispielsweise am Morgen, am Mittag oder am Abend eingenommen werden. Bevorzugt ist eine q.d. oder b.i.d. Verabreichung bei Herzschwäche.

In einer Ausführungsform der Erfindung wird der NEP Inhibitor in Einheitsformen beispielsweise Tabletten oder Kapseln verabreicht, die beispielsweise 20 mg bis 800 mg, vorzugsweise 50 mg bis 700 mg, bevorzugter 100 mg bis 600 mg und noch bevorzugter 100 mg bis 300 mg umfassen, die einmal am Tag verabreicht werden.

Die oben beschriebenen Dosierungen können im Rahmen der Erfindung als Fixed-Dose-Kombination formuliert werden, worin die bevorzugten Einheitsformen Tabletten oder Kapseln sein können.

Bei einer bevorzugten Ausführungsform der Erfindung beträgt die Dosierung des sGC Stimulators etwa 2.5 -20 mg od, die Dosierung von Valsartan etwa 25 mg bid und die Dosierung des NEP-Inhibitors beträgt etwa 25 mg bid, ebenfalls bevorzugt beträgt die Dosierung des sGC Stimulators und/oder sGC Aktivators etwa 2.5-20 mg od, die Dosierung von Valsartan etwa 50 mg bid und die Dosierung des NEP-Inhibitors beträgt etwa 50 mg bid und ebenfalls bevorzugt beträgt die Dosierung von des sGC Stimulators etwa 2.5-20 mg od, die Dosierung von Valsartan etwa 100 mg bid und die Dosierung des NEP-Inhibitors beträgt etwa 100 mg bid.

Bei einer bevorzugten Ausführungsform der Erfindung beträgt die Dosierung des sGC Stimulators etwa 5-10 mg od, die Dosierung von Valsartan etwa 25 mg bid und die Dosierung des NEP-Inhibitors beträgt etwa 25 mg bid, ebenfalls bevorzugt beträgt die Dosierung des sGC Stimulators etwa 5-10 mg od, die Dosierung von Valsartan etwa 50 mg bid und die Dosierung des NEP-Inhibitors beträgt etwa 50 mg bid und ebenfalls bevorzugt beträgt die Dosierung von des sGC Stimulators etwa 5-10 mg od, die Dosierung von Valsartan etwa 100 mg bid und die Dosierung des NEP-Inhibitors beträgt etwa 100 mg bid.

Bei einer bevorzugten Ausführungsform der Erfindung beträgt die Dosierung des sGC Stimulators etwa 2.5-5 mg od, die Dosierung von Valsartan etwa 25 mg bid und die Dosierung des NEP-Inhibitors beträgt etwa 25 mg bid, ebenfalls bevorzugt beträgt die Dosierung des sGC Stimulators etwa 2.5-5 mg od, die Dosierung von Valsartan etwa 50 mg bid und die Dosierung des NEP-Inhibitors beträgt etwa 50 mg bid und ebenfalls bevorzugt beträgt die Dosierung von des sGC Stimulators etwa 2.5-5 mg od, die Dosierung von Valsartan etwa 100 mg bid und die Dosierung des NEP-Inhibitors beträgt etwa 100 mg bid.

Bei einer bevorzugten Ausführungsform der Erfindung beträgt die Dosierung des sGC Stimulators etwa 10-20 mg od, die Dosierung von Valsartan etwa 25 mg bid und die Dosierung des NEP-Inhibitors beträgt etwa 25 mg bid, ebenfalls bevorzugt beträgt die Dosierung des sGC Stimulators etwa 10-20 mg od, die Dosierung von Valsartan etwa 50 mg bid und die Dosierung des NEP-Inhibitors beträgt etwa 50 mg bid und ebenfalls bevorzugt beträgt die Dosierung von des sGC Stimulators etwa 10-20 mg od, die Dosierung von Valsartan etwa 100 mg bid und die Dosierung des NEP-Inhibitors beträgt etwa 100 mg bid.

Bei einer weiteren bevorzugten Ausführungsform der Erfindung beträgt die Dosierung des sGC Stimulators etwa 2.5-20 mg od und die Dosierung von LCZ696 etwa 50 mg bid, ebenfalls bevorzugt beträgt die Dosierung von des sGC Stimulators etwa 5-10 mg od und die von LCZ696 etwa 100 mg bid und ebenfalls bevorzugt beträgt die Dosierung von des sGC Stimulators etwa 2.5-5 mg od und die Dosierung von LCZ696 etwa 200 mg bid.

Bei einer weiteren bevorzugten Ausführungsform der Erfindung beträgt die Dosierung des sGC Stimulators etwa 10-20 mg od und die Dosierung von LCZ696 etwa 50 mg bid, ebenfalls bevorzugt beträgt die Dosierung von des sGC Stimulators etwa 10-20 mg od und die von LCZ696 etwa 100 mg bid und ebenfalls bevorzugt beträgt die Dosierung von des sGC Stimulators etwa 10-20 mg od und die Dosierung von LCZ696 etwa 200 mg bid.

Bei einer weiteren bevorzugten Ausführungsform der Erfindung beträgt die Dosierung des sGC Stimulators etwa 2.5-5 mg od und die Dosierung von LCZ696 etwa 50 mg bid, ebenfalls bevorzugt beträgt die Dosierung von des sGC Stimulators etwa 2.5-5 mg od und die von LCZ696 etwa 100 mg bid und ebenfalls bevorzugt beträgt die Dosierung von des sGC Stimulators etwa 2.5-5 mg od und die Dosierung von LCZ696 etwa 200 mg bid.

Bei einer weiteren bevorzugten Ausführungsform der Erfindung beträgt die Dosierung des sGC Stimulators etwa 5-10 mg od und die Dosierung von LCZ696 etwa 50 mg bid, ebenfalls bevorzugt beträgt die Dosierung von des sGC Stimulators etwa 5-10 mg od und die von LCZ696 etwa 100 mg bid und ebenfalls bevorzugt beträgt die Dosierung von des sGC Stimulators etwa 5-10 mg od und die Dosierung von LCZ696 etwa 200 mg bid.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Experimenteller Teil

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Bewertung der physiologischen Wirksamkeit

Die Eignung der erfindungsgemäßen Kombinationen zur Behandlung von kardiovaskulären Erkrankungen kann in folgenden Assaysystemen gezeigt werden:

### Radiotelemetrische Messung von Blutdruck und Herzfrequenz an wachen Ratten

Für die im Folgenden beschriebenen Messungen an wachen Ratten (Stämme Wistar Unilever/WU oder Spontaneous Hypertensive Rat/SHR) wurde ein im Handel erhältliches Telemetriesystem der Firma Data Sciences International DSI, USA, eingesetzt. Das System besteht aus 3 Hauptkomponenten: (1) Implantierbare Sender (PhysioTel^{®} Telemetrietransmitter), (2) Empfänger (PhysioTel^{®} Receiver), die über einen Multiplexer (DSI Data Exchange Matrix) mit einem (3) Datenakquisitionscomputer verbunden sind. Die Telemetrieanlage ermöglicht eine kontinuierliche Erfassung von Blutdruck, Herzfrequenz und Körperbewegung an wachen Tieren in ihrem gewohnten Lebensraum.

Die Untersuchungen werden an ausgewachsenen weiblichen Ratten mit einem Körpergewicht von > 200 g durchgeführt. Die Versuchstiere werden nach Senderimplantation einzeln in Makrolon^{®}-Käfigen Typ III gehalten. Sie haben freien Zugang zu Standardfutter und Wasser. Der Tag/Nacht-Rhythmus im Versuchslabor wird durch Wechsel der Raumbeleuchtung eingestellt.

### Senderimplantation:

Die eingesetzten Telemetriesender (z.B. PA-C40, HD-S10, DSI) werden den Versuchstieren mindestens 14 Tage vor dem ersten Versuchseinsatz unter aseptischen Bedingungen chirurgisch implantiert.

Zur Implantation werden die nüchternen Tiere mit Isofluran narkotisiert (IsoFlo^{®}, Abbott, Einleitung 5%, Erhaltung 2%) und an der Bauchseite weiträumig rasiert und desinfiziert. Nach Eröffnung des Bauchraumes entlang der *Linea alba* wird der flüssigkeitsgefüllte Messkatheter des Systems oberhalb der Bifurkation nach cranial in die *Aorta descendens* eingesetzt und mit Gewebekleber (Vetbond^{™}, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum (Ursocyclin^{®} 10%, 60 mg/kg s.c., 0.06 ml/100 g Körpergewicht, Serumwerk Bernburg AG, Deutschland) sowie ein Analgetikum (Rimadyl^{®}, 4 mg/kg s.c., Pfizer, Deutschland) verabreicht.

### Substanzen und Lösungen:

Wenn nicht anders beschrieben, werden die zu untersuchenden Substanzen jeweils einer Gruppe von Tieren (N=6) oral verabreicht. Entsprechend einem Applikationsvolumen von 2 ml/kg Körpergewicht werden die Testsubstanzen in geeigneten Lösungsmittelgemischen gelöst. Eine Lösungsmittel-behandelte Gruppe von Tieren wird als Kontrolle eingesetzt.

### Versuchsablauf:

Die Telemetrie-Messeinrichtung ist für 24 Tiere konfiguriert.

Den in der Anlage lebenden instrumentierten Ratten ist jeweils eine eigene Empfangsantenne zugeordnet (RPC-1 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest^{™} A.R.T. for Windows, DSI oder Ponemah, DSI) online erfasst und entsprechend aufgearbeitet werden.

Im Standardablauf werden über je 10 Sekunden Dauer gemessen: (1) systolischer Blutdruck (SBP), (2) diastolischer Blutdruck (DBP), (3) arterieller Mitteldruck (MAP), (4) Herzfrequenz (HR) und (5) Aktivität (ACT). Diese Parameter werden im Anschluss an die Applikation über 24 Stunden gemessen.

Die Messwerterfassung wird rechnergesteuert in 5 Minuten-Abständen wiederholt. Die als Absolutwert erhobenen Quelldaten werden im Diagramm mit dem aktuell gemessenen Barometerdruck (Ambient Pressure Reference Monitor, APR-1, DSI) korrigiert.

### Auswertung:

Nach Versuchsende werden die erhobenen Einzeldaten mit der Analysis-Software (Dataquest^{™} A.R.T. 4.1 Analysis oder Ponemah, DSI) sortiert. Als Leerwert wird der Zeitpunkt 2 Stunden vor SubstanzApplikation angenommen.

Die Daten werden über eine voreinstellbare Zeit durch Mittelwertbestimmung geglättet (30 Minuten-Mittelwert).

### Literatur:

K. Witte, K. Hu, J. Swiatek, C. Müssig, G. Ertl und B. Lemmer, Experimental heart failure in rats: effects on cardiovascular circadian rhythms and on myocardial β-adrenergic signaling, Cardiovasc. Res. 47 (2): 203-405, 2000.

### Langzeitstudie in L-NAME behandelten transgenen Ren2-Ratten

Die kardiovaskuläre Wirkung nach Gabe eines sGC Modulators, Neprilysin Inhibitors, Angiotensin All-Antagonisten, und Doppel- und Dreifachkombination hiervon werden durch die Bestimmung der Langzeiteffekte auf hämodynamische und hormonelle Parameter in einem Hoch-Renin, Niedrig-NO Bluthochdruckmodell in Ratten gezeigt.

Männliche transgene Ren2-Ratten (TGR(mRen2)27) werden vor Ort gezüchtet und unter kontrollierten Licht- und Temperaturkonditionen gehalten. Ab einem Alter von 10-20 Wochen werden die Tiere in verschiedene Gruppen randomisiert. Die Kontrollgruppe erhalten ein Placebo, die Testgruppen sGC Modulatoren, Neprilysin Inhibitoren, Angiotensin All-Antagonisten, und Doppel- und Dreifachkombination hiervon für 4-10 Wochen. Die Ratten der Placebo und Behandlungsgruppen erhalten zudem 30-100 mg/l L-NAME über das Trinkwasser. Die Testsubstanzen werden als Suspension in einem Gemisch aus Transcutol/Cremophor/Wasser (10/20/70=V/V/V) oder als Tylosesuspension p.o. verabreicht. Systolischer Blutdruck und die Herzfrequenz werden wöchentlich mit Hilfe der "tail-cuff" Methode in wachen Tieren in Käfigen bei konstanter Temperatur von 37°C gemessen. Urinsammlungen erfolgen an Tag 0, während und am Ende des Experiments in Metabolismus-Käfigen und Proteinurie, urinale Elektrolytausscheidung werden bestimmt. Beim Studienabschluss werden der linksventrikuläre Druck und die kardiale Kontraktionsfähigkeit unter Anästhesie gemessen. Zuletzt werden die Tiere durch Dekapitation getötet und Blutproben entnommen. Plasma- und Urinparameter werden biochemisch bestimmt z.B. ANP (RIA Kit RK 005-24, Phoenix Pharmaceuticals, Inc., USA), cGMP (RIA Kit RE29075, IBL International GmbH, Deutschland), Renin, Angiotensin I (RIA Kit CA-1533, DiaSorin S.p.A., Italien) und Aldosteron (P2714, DiaSorin S.p.A., Italien). Organe z.B. Niere, Herz, Lunge u.s.w. werden entnommen und Genexpressionsprofile als auch histopathologische Daten erhoben.

### Hypertensives Herzinsuffizenz-Modell in Hunden

Die kardiovaskuläre Wirkung nach Gabe eines sGC Modulators (sGC Stimulator oder sGC Aktivator), Neprilysin Inhibitors, Angiotensin All-Antagonisten, und Doppel- und Dreifachkombination hiervon werden in einem Hundemodell einer "milden" systolischen Herzinsuffizenz, verursacht durch Angiotensin All Antagonisten-induzierte akute Hypertension evaluiert. Ein tierexperimentelles Modell einer linksventrikulären Dysfunktion wird durch rechtsventrikuläres "Tachypacing" bei 180 bpm für 10 Tage ausgelöst. Die experimentelle Durchführung dieses Modells wurde ausführlich beschrieben. [Redfield et al., Circulation 1993;87:2016-2022]

### Hypertensive "renal wrap" Hunde

Die Wirkung eines sGC Modulators (sGC Stimulator oder sGC Aktivator), Neprilysin Inhibitors, Angiotensin All-Antagonisten, und Doppel- und Dreifachkombination hiervon auf Blutdruck und Herzfrequenz werden im hypertensiven "renal wrap" Hundemodell geprüft. Im Modell wird eine Niere mit Seide eingewickelt, während die zweite Niere einer Okklusion der Hauptnierenarterie unterzogen wird. [Page et al., Science 1939; 89: 2307-2308; Goldblatt et al., Proc Natl Acad Sci 1976; 73: 1722-1724]. Ungefähr 4 Wochen nach dem Eingriff, entwickeln die Hunde einen stabilen Bluthochdruck.

### Ergebnisse:

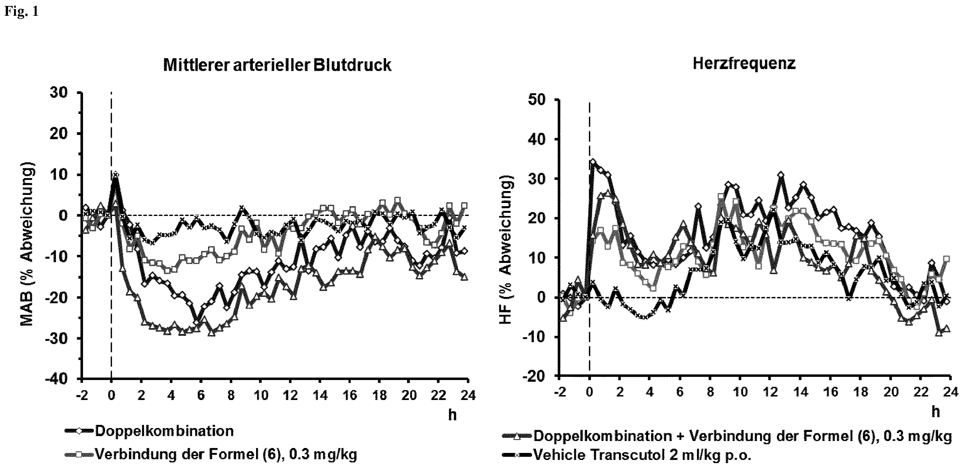

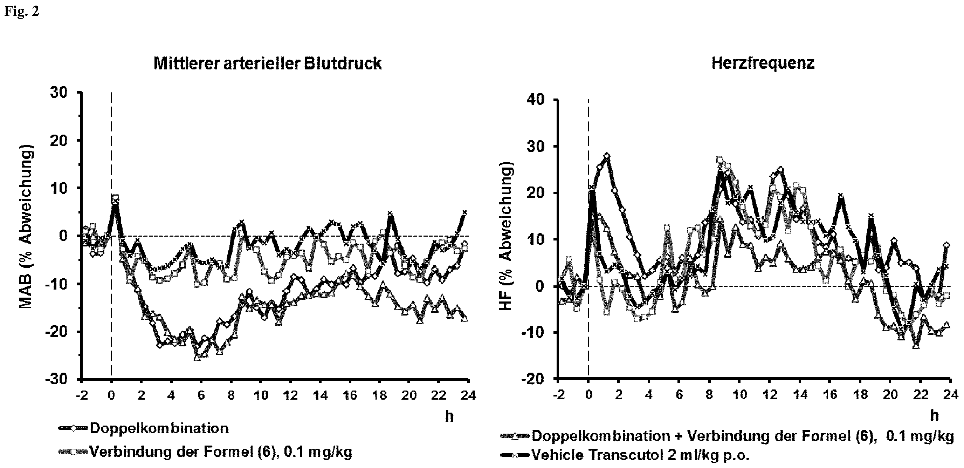

Die Ergebnisse sind für die Verbindung der Formel (6) in Dreifachkombination mit einem Neprilysin Inhibitor und einem Angiotensin All-Antagonisten im Vergleich zu der alleinigen Gabe der Verbindung der Formel (6) und einer Doppelkombination aus einem Neprilysin Inhibitor und einem Angiotensin All-Antagonisten in den Abbildungen 1 und 2 dargestellt.

Die Verbindung der Formel (6) zeigt keine hämodynamischen Effekte (Blutdruck, Herzfrequenz) bei einer per oralen Dosierung von 0.1 mg/kg; mit 0.3 mg/kg wurde eine Senkung des mittleren arterieller Blutdrucks um ca. -10% und ein transienter Anstieg der Herzfrequenz beobachtet. Die als Doppelkombination per oral applizierten Vergleichssubstanzen zeigen in der untersuchten Dosierung eine Senkung des mittleren arteriellen Blutdrucks um ca. -20% mit assoziierter Reflextachykardie. Der Zusatz von 0.1 mg/kg der Verbindung der Formel (6) zur Doppelkombination führt im Vergleich zur Doppelkombination alleine bei gleicher Senkung des mittleren arteriellen Blutdrucks zu einem verringerten reflektorischen Anstieg der Herzfrequenz.
Die Kombination der Verbindung der Formel (6) in einer Dosierung von 0.3 mg/kg mit einem Neprilysin Inhibitor und einem Angiotensin All-Antagonisten zeigt einen zusätzlichen Effekt auf die Blutdrucksenkung. Überraschenderweise war in dem Versuch der Dreifachkombination ein nicht additiver Effekt auf die Herzfrequenz zu beobachten (kein additiver kompensatorischer Effekt bzw. Reflextachykardie). In der Doppel- und Dreifachkombination wird 30 mg/kg Sacubitril und 10 mg/kg Valsartan eingesetzt.

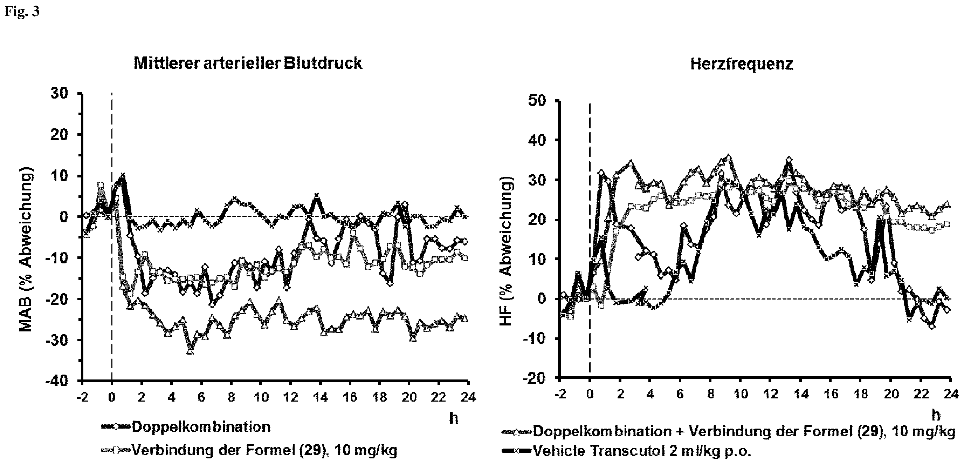

Die Ergebnisse sind für die Verbindung der Formel (29) in Dreifachkombination mit einem Neprilysin Inhibitor und einem Angiotensin All-Antagonisten im Vergleich zu der alleinigen Gabe der Verbindung der Formel (29) und einer Doppelkombination aus einem Neprilysin Inhibitor und einem Angiotensin All-Antagonisten in den Abbildungen 3, 4 und 5 dargestellt.

Die Verbindung der Formel (29) zeigt bei einer per oralen Dosierung von 3 mg/kg und 10 mg/kg eine Senkung des mittleren arterieller Blutdrucks um ca. -10% bzw. 15% und eine assoziierte Reflextachykardie. Die Verbindung der Formel (29) zeigt bei einer per oralen Dosierung von 1 mg/kg eine Reflextachykardie. Die als Doppelkombination per oral applizierten Vergleichssubstanzen zeigen in der untersuchten Dosierung eine Senkung des mittleren arteriellen Blutdrucks um ca. -15%. Die dreifache Kombination der Verbindung der Formel (29) mit einem Neprilysin Inhibitor und einem Angiotensin All-Antagonisten zeigen einen Effekt auf die Blutdrucksenkung. In der Doppel- und Dreifachkombination wird 30 mg/kg Sacubitril und 10 mg/kg Valsartan eingesetzt.

### Beschreibung der Abbildungen:

Fig. 1: B-x) Mittlerer arterieller Blutdruck und Herzfrequenz in % Abweichung gegen die Zeit [h] nach Substanzapplikation; Verbindung der Formel (6), 0.3 mg/kg p.o.; Doppelkombination: 30 mg/kg Sacubitril und 10 mg/kg Valsartan p.o., Dreifachkombination: Verbindung der Formel (6), 0.3 mg/kg, 30 mg/kg Sacubitril und 10 mg/kg Valsartan p.o..
Fig. 2: B-x) Mittlerer arterieller Blutdruck und Herzfrequenz in % Abweichung gegen die Zeit [h] nach Substanzapplikation; Verbindung der Formel (6), 0.1 mg/kg p.o.; Doppelkombination: 30 mg/kg Sacubitril und 10 mg/kg Valsartan p.o., Dreifachkombination: Verbindung der Formel (6), 0.1 mg/kg, 30 mg/kg Sacubitril und 10 mg/kg Valsartan p.o..
Fig. 3: B-x) Mittlerer arterieller Blutdruck und Herzfrequenz in % Abweichung gegen die Zeit [h] nach Substanzapplikation; Verbindung der Formel(29), 10 mg/kg p.o.; Doppelkombination: 30 mg/kg Sacubitril und 10 mg/kg Valsartan p.o., Dreifachkombination: Verbindung der Formel (29), 10 mg/kg, 30 mg/kg Sacubitril und 10 mg/kg Valsartan p.o..

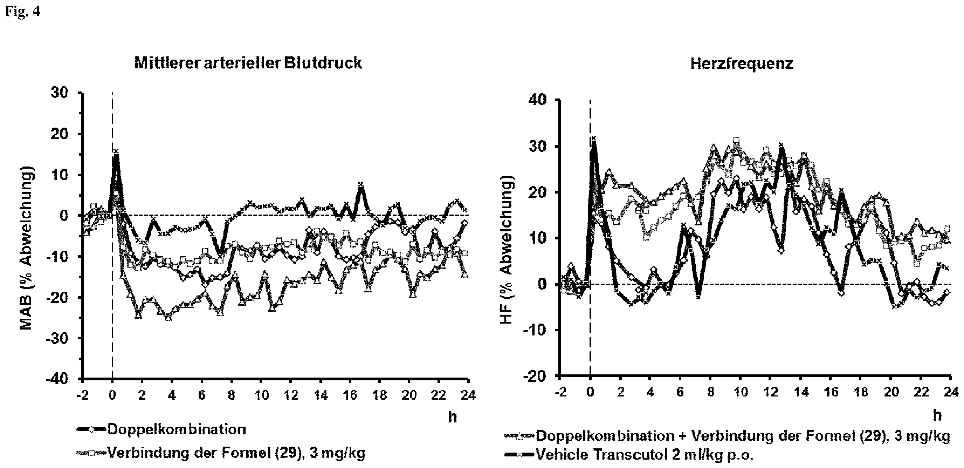

Fig. 4: B-x) Mittlerer arterieller Blutdruck und Herzfrequenz in % Abweichung gegen die Zeit [h] nach Substanzapplikation; Verbindung der Formel (29), 3 mg/kg p.o.; Doppelkombination: 30 mg/kg Sacubitril und 10 mg/kg Valsartan p.o., Dreifachkombination: Verbindung der Formel (29), 3 mg/kg, 30 mg/kg Sacubitril und 10 mg/kg Valsartan p.o..

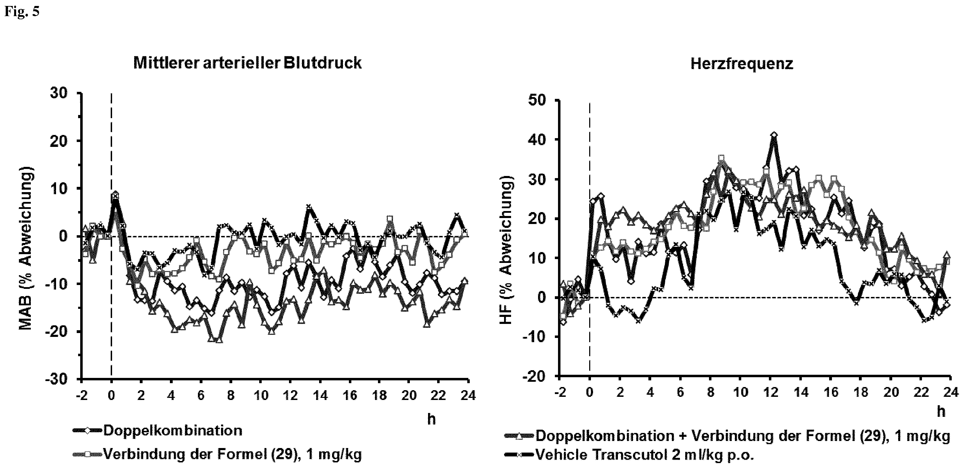

Fig. 5: B-x) Mittlerer arterieller Blutdruck und Herzfrequenz in % Abweichung gegen die Zeit [h] nach Substanzapplikation; Verbindung der Formel (29), 1 mg/kg p.o.; Doppelkombination: 30 mg/kg Sacubitril und 10 mg/kg Valsartan p.o., Dreifachkombination: Verbindung der Formel (29), 1 mg/kg, 30 mg/kg Sacubitril und 10 mg/kg Valsartan p.o.

## Patentansprüche

1. Kombinationen enthaltend den sGC Stimulator der Formel 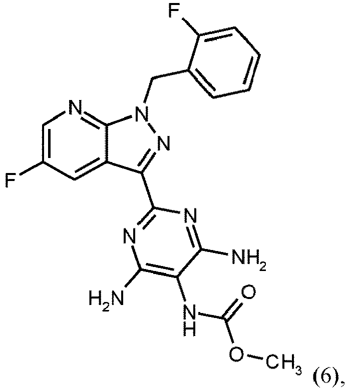 Sacubitril und Valsartan sowie jeweils die Salze, Solvate und Solvate der Salze davon.

2. Kombinationen gemäß Anspruch 1 enthaltend Trinatrium[3-((1*S*,3*R*)-1-biphenyl-4-ylmethyl-3-ethoxycarbonyl-1-butylcarbamoyl)propionat-(*S*)-3'-methyl-2'-(pentanoyl{2"-(tetrazol-5-ylat)biphenyl-4'-ylmethyl}amino)butyrat]hemipentahydrat sowie jeweils die Salze, Solvate und Solvate der Salze davon.

3. Kombination gemäß einem der Ansprüche 1 oder 2 zur Anwendung in einem Verfahren zur Behandlung und/oder Prophylaxe von Herz-Kreislauf-Erkrankungen, renalen Erkrankungen, Lungenerkrankungen, sowie zur Behandlung und/oder Prophylaxe von fibrotischen Erkrankungen.

4. Arzneimittel enthaltend eine Kombination gemäß einem der Ansprüche 1 oder 2 in Kombination mit einem inerten, nicht-toxischen, pharmazeutisch geeigneten Hilfsstoff.

5. Arzneimittel enthaltend eine Kombination gemäß einem der Ansprüche 1 oder 2 in Kombination mit einem oder mehreren weiteren Wirkstoffen ausgewählt aus der Gruppe bestehend aus ACE-Inhibitoren, Renin-Inhibitoren, Beta-Blocker, Acetylsalicylsäure, Diuretika, Calcium-Antagonisten, Statine, Digitalis (Digoxin)-Derivate, Calcium-Sensitizer, Nitrate sowie Antithrombotika.

6. Arzneimittel enthaltend eine Kombination gemäß einem der Ansprüche 1 oder 2 zur Anwendung in einem Verfahren zur Behandlung und/oder Prophylaxe von Herz-Kreislauf-Erkrankungen, renalen Erkrankungen, Lungenerkrankungen, sowie zur Behandlung und/oder Prophylaxe von fibrotischen Erkrankungen.

7. Kit umfassend eine pharmazeutische Zusammensetzung enthaltend den sGC Stimulator der Formel 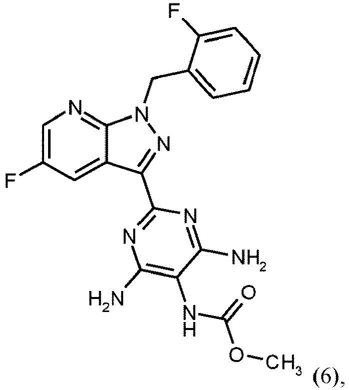 und eine pharmazeutische Zusammensetzung enthaltend Valsartan und N-(3-Carboxy-1-oxopropyl)-(4*S*)-(p-phenylphenyl-methyl)-4-amino-2*R*-methylbutansäure oder einen Ester davon.

8. Kit gemäß Anspruch 7 umfassend eine pharmazeutische Zusammensetzung enthaltend den sGC Stimulator der Formel 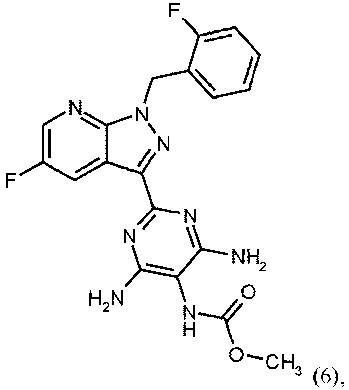 und eine pharmazeutische Zusammensetzung enthaltend Trinatrium-[3-((1*S*,3*R*)-1-biphenyl-4-ylmethyl-3-ethoxycar-bonyl-1-butylcarbamoyl)propionat-(*S*)-3'-methyl-2'-(pentanoyl{2"-(tetrazol-5-ylat)biphenyl-4'-ylmethyl}amino)butyrat]-hemipentahydrat.

## Claims

1. Combinations comprising the sGC stimulator of the formula 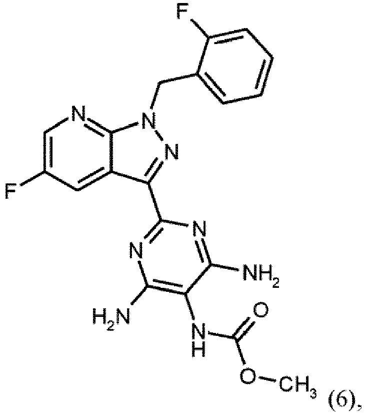 sacubitril and valsartan and also in each case the salts, solvates and solvates of the salts thereof.

2. Combinations according to Claim 1 comprising trisodium [3-((1*S*,3*R*)-1-biphenyl-4-ylmethyl-3-ethoxycarbonyl-1-butylcarbamoyl)propionate-(*S*)-3'-methyl-2'-(pentanoyl{2"-(tetrazol-5-ylate)biphenyl-4'-ylmethyl}amino)butyrate] hemipentahydrate and also in each case the salts, solvates and solvates of the salts thereof.

3. Combination according to either of Claims 1 or 2 for use in a method for the treatment and/or prophylaxis of cardiovascular disorders, renal disorders, lung disorders, and also for the treatment and/or prophylaxis of fibrotic disorders.

4. Medicament comprising a combination according to either of Claims 1 or 2 in combination with an inert, non-toxic, pharmaceutically suitable excipient.

5. Medicament comprising a combination according to either of Claims 1 or 2 in combination with one or more further active ingredients selected from the group consisting of ACE inhibitors, renin inhibitors, beta blockers, acetylsalicylic acid, diuretics, calcium antagonists, statins, digitalis (digoxin) derivatives, calcium sensitizers, nitrates and antithrombotics.

6. Medicament comprising a combination according to either of Claims 1 or 2 for use in a method for the treatment and/or prophylaxis of cardiovascular disorders, renal disorders, lung disorders, and also for the treatment and/or prophylaxis of fibrotic disorders.

7. Kit comprising a pharmaceutical composition comprising the sGC stimulator of the formula 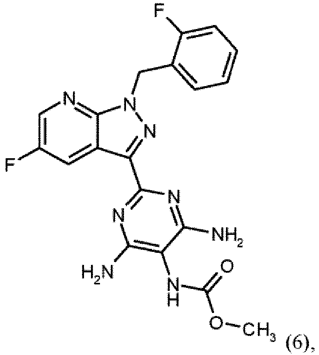 and a pharmaceutical composition comprising valsartan and N-(3-carboxy-1-oxopropyl)-(4*S*)-(p-phenylphenylmethyl)-4-amino-2*R*-methylbutanoic acid or an ester thereof.

8. Kit according to Claim 7 comprising a pharmaceutical composition comprising the sGC stimulator of the formula 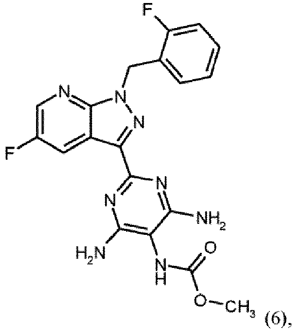 and a pharmaceutical composition comprising trisodium [3-((1*S*,3*R*)-1-biphenyl-4-ylmethyl-3-ethoxycarbonyl-1-butylcarbamoyl)propionate-(*S*)-3'-methyl-2'-(pentanoyl{2"-(tetrazol-5-ylate)biphenyl-4'-ylmethyl}amino)butyrate] hemipentahydrate.

## Revendications

1. Combinaisons contenant le stimulateur de GCs de formule 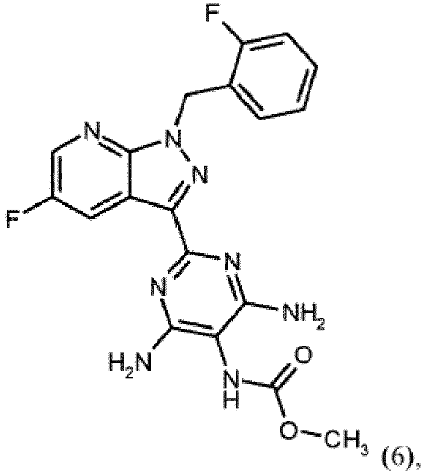 du sacubitril et du valsartan ainsi que, à chaque fois, les sels, les solvates et les solvates des sels correspondants.

2. Combinaisons selon la revendication 1 contenant de l'hémipentahydrate de [3-((1*S*,3*R*)-1-biphényl-4-ylméthyl-3-éthoxycarbonyl-1-butylcarbamoyl)propionate-(*S*)-3'-méthyl-2'-(pentanoyl{2"-(tétrazol-5-ylate)biphényl-4'-ylméthyl}amino)butyrate] trisodique ainsi que, à chaque fois, les sels, les solvates et les solvates des sels correspondants.

3. Combinaison selon l'une quelconque des revendications 1 ou 2 pour une application dans un procédé pour le traitement et/ou la prophylaxie de maladies cardiovasculaires, de maladies rénales, de maladies des poumons, ainsi que pour le traitement et/ou la prophylaxie de maladies fibrotiques.

4. Médicament contenant une combinaison selon l'une quelconque des revendications 1 ou 2 en combinaison avec un excipient inerte, non toxique, pharmaceutiquement approprié.

5. Médicament contenant une combinaison selon l'une quelconque des revendications 1 ou 2 en combinaison avec un ou plusieurs principes actifs supplémentaires choisis dans le groupe constitué par des inhibiteurs de l'ECA, des inhibiteurs de rénine, des bêtabloquants, l'acide acétylsalicylique, un diurétique, des antagonistes du calcium, une statine, des dérivés de digitaline (digoxine), des sensibilisateurs du calcium, des nitrates ainsi que des antithrombotiques.

6. Médicament contenant une combinaison selon l'une quelconque des revendications 1 ou 2 pour une application dans un procédé pour le traitement et/ou la prophylaxie de maladies cardiovasculaires, de maladies rénales, de maladies des poumons, ainsi que pour le traitement et/ou la prophylaxie de maladies fibrotiques.

7. Kit comprenant une composition pharmaceutique contenant le stimulateur de GCs de formule 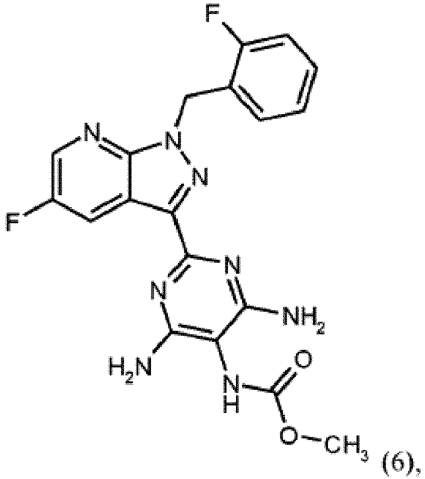 et une composition pharmaceutique contenant du valsartan et de l'acide N-(3-carboxy-1-oxopropyl)-(4*S*)-(p-phénylphényl-méthyl)-4-amino-2*R-*méthylbutyrique ou un ester correspondant.

8. Kit selon la revendication 7 comprenant une composition pharmaceutique contenant le stimulateur de GCs de formule 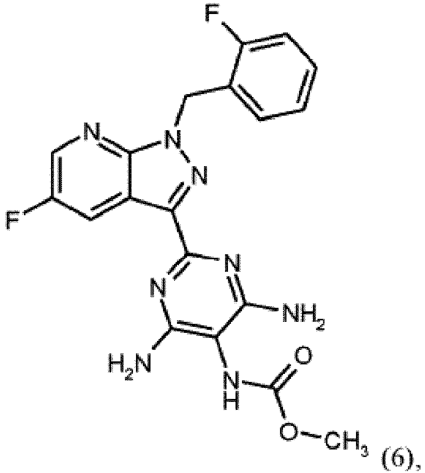 et une composition pharmaceutique contenant de l'hémipentahydrate de [3-((1*S*,3*R*)-1-biphényl-4-ylméthyl-3-éthoxycarbonyl-1-butylcarbamoyl)propionate-(*S*)-3'-méthyl-2'-(pentanoyl{2"-(tétrazol-5-ylate)biphényl-4'-ylméthyl}amino)butyrate] trisodique.
